# NOUVEAU FASCICULE DE BREVET EUROPEEN

(11) **EP 3 212 200 B2**
(45) Date de publication et mention de la décision concernant l'opposition: **24.12.2025**
(45) Mention de la délivrance du brevet: 20.01.2021
(21) Numéro de dépôt: 15798211.7
(22) Date de dépôt: 30.10.2015
(51) Int. Cl.: A61K 31/7072, A61P 25/28, A61P 25/00, A61P 27/00

(54) **UTILISATION D'UN INHIBITEUR DE TRANSCRIPTASE INVERSE DANS LA PREVENTION ET LE TRAITEMENT DES MALADIES DEGENERATIVES**
VERWENDUNG VON REVERSE TRANSCRIPTASEHEMMER FÜR DIE BENANDLUNG VON DEGENERATIVEN KRANKHEITEN
USE OF AN INVERSE TRANSCRIPTASE INHIBITOR FOR THE TREATMENT AND PREVENTION OF DEGENERATIVE DISEASES

(30) Priorité: 31.10.2014 FR 1460535
(43) Date de publication de la demande: 06.09.2017
(73) Titulaire: Centre National de la Recherche Scientifique, 75016 Paris (FR); College de France, 75231 Paris Cedex 05 (FR); INSERM - Institut National de la Santé et de la Recherche Médicale, 75013 Paris (FR); Sorbonne Université, 75006 Paris 6 (FR)
(72) Inventeur: PROCHIANTZ, Alain, F-75006 Paris (FR); FUCHS, Julia, F-93500 Pantin (FR); JOSHI, Rajiv, F-91300 Massy (FR); BLAUDIN DE THE, François Xavier, F-75007 Paris (FR); REKAIK, Hocine, F-77000 Melun (FR); MASSIANI-BEAUDOIN, Olivia, F-91230 Montgeron (FR)
(74) Mandataire: Gevers & Orès
(86) Numéro de dépôt international: PCT/IB2015/058404
(87) Numéro de publication internationale: WO 2016/067265

(56) Documents cités:
- WO-A1-00/50043
- WO-A1-2009/153009
- WO-A1-2009/153009
- DE-A1- 4 307 883
- KR-A- 20100 073 528
- US-A1- 2014 113 952
- US-A1- 2015 018 297
- CHU, S.C. ET AL.: "Inhibitory effects of flavonoids on Moloney murine leukemia virus reverse transcriptase activity", J NAT PROD., vol. 55, no. 2, 1992, pages 179 - 83, XP002739893, DOI: 10.1021/np50080a005
- SONNIER, L. ET AL.: "Progressive Loss of Dopaminergic Neurons in the Ventral Midbrain of Adult Mice Heterozygote for Engrailed1", THE JOURNAL OF NEUROSCIENCE, vol. 27, no. 5, 31 January 2007 (2007-01-31), pages 1063 - 1071, XP002445228, DOI: 10.1523/JNEUROSCI.4583-06.2007
- AHN MI-JEONG ET AL: "Inhibition of HIV-1 reverse transcriptase and protease by phlorotannins from the brown alga Ecklonia cava.", BIOLOGICAL & PHARMACEUTICAL BULLETIN APR 2004, vol. 27, no. 4, April 2004 (2004-04-01), pages 544 - 547, XP002739892, ISSN: 0918-6158
- CHU S C ET AL: "Inhibitory effects of flavonoids on Moloney murine leukemia virus reverse transcriptase activity.", JOURNAL OF NATURAL PRODUCTS FEB 1992, vol. 55, no. 2, February 1992 (1992-02-01), pages 179 - 183, XP002739893, ISSN: 0163-3864
- WESTARP M E ET AL: "Antiretroviral therapy in sporadic adult amyotrophic lateral sclerosis.", NEUROREPORT JUN 1993, vol. 4, no. 6, June 1993 (1993-06-01), pages 819 - 822, XP009184352, ISSN: 0959-4965
- WESTARP M E ET AL: "AMYOTROPHIC LATERAL SCLEROSIS AN ANIGMATIC DISEASE WITH B-CELLULAR AND ANTI-RETROVIRAL IMMUNE RESPONSE", EUROPEAN JOURNAL OF MEDICINE, PRESSE MEDICALE, PARIS, FR, vol. 2, no. 6, 1 June 1993 (1993-06-01), pages 327 - 332, XP000675737, ISSN: 1165-0478

## Description

La présente invention concerne l'utilisation d'un inhibiteur de transcriptase inverse dans la prévention et le traitement des maladies dégénératives.

Une caractéristique de la plupart des maladies dégénératives, en particulier neurodégénératives, est qu'elles se manifestent tard dans la vie. Cela est vrai de leurs formes sporadiques mais aussi génétiques, comme cela est illustré par la maladie de Parkinson, la maladie d'Alzheimer et même la maladie monogénique de Huntington. Cela suggère que le vieillissement rend les cellules plus sensibles, même si on ne peut exclure que les mutations elles-mêmes accélèrent ce vieillissement.

Le stress oxydatif qui mime un vieillissement accéléré génère des dérivés réactifs de l'oxygène (ROS pour *Reactive Oxygen Species*) qui sont toxiques, en particulier au niveau du génome où les protéines de la chromatine et les bases de l'ADN sont soumises à une oxydation (Vijg, J., and Suh, Y., 2013). Les dérivés réactifs de l'oxygène peuvent induire des cassures simple-brin et double-brin (DSB pour *Double-Strand Break*) de l'ADN, activant ainsi les systèmes de réponse aux dommages faits à l'ADN (O'Sullivan and Karlseder, 2012; Marteijn *et al.,* 2014). Par exemple, un article récent montre que chez la souris l'activité neuronale et donc la synthèse d'ATP et de dérivés actifs de l'oxygène sont accompagnées de façon physiologique par la formation de cassures double-brin de l'ADN qui sont rapidement réparées chez les souris de type sauvage, contrairement aux souris J20, modèle de la maladie d'Alzheimer (Suberbielle *et al.,* 2013).

Les longs éléments nucléaires intercalés LINEs (LINE pour *Long Interspersed Nuclear Elements*) font partie des séquences répétées dispersées au sein de l'ADN. Les séquences LINE actives sont des éléments transposables sans longue séquence terminale répétée (LTR pour *Long Terminal Repeat*) capables de réplication autonome, ce qui leur permet de se dupliquer et de s'insérer à d'autres sites dans le génome. Les LINE codent pour un ARN polycistronique avec 2 phases ouvertes de lecture, ORF1 et ORF2. ORF2 encode une transcriptase inverse (RT pour Reverse Transcriptase) qui copie l'ARN du LINE en ADN et une endonucléase qui permet l'insertion de cet ADN dans le génome à travers la formation d'une cassure double-brin, et qui -au passage-introduit une mutation dans le génome qui peut s'avérer délétère.

Les inventeurs ont montré que le stress oxydatif qui mime un vieillissement accéléré, induit la formation de cassures double-brin (DSB) de l'ADN, une déstructuration de l'hétérochromatine et l'expression des gènes d'éléments transposables (LINE). Ils ont proposé que l'expression des LINE induite par le stress oxydatif est à l'origine, au moins en partie, de la formation des DSB et démontré que la formation de ces DSB est diminuée en présence d'un inhibiteur de transcriptase inverse (ou reverse transcriptase).

Sur la base de ces observations, les inventeurs proposent d'utiliser les inhibiteurs de transcriptase inverse dans la prévention et le traitement des maladies dégénératives.

L'objet de la présente invention est défini par les revendications 1 à 3.

L'invention concerne un inhibiteur de transcriptase inverse destiné à être utilisé dans la prévention ou le traitement d'une maladie dégénérative choisie parmi la maladie de Parkinson et la maladie d'Alzheimer, ledit inhibiteur de transcriptase inverse étant choisi parmi :
- les inhibiteurs nucléosidiques,
- l'Efavirenz (EFV),
- la Névirapine (NVP),
- la Delavirdine (DLV),
- l'Etravirine, et
- la Rilvipirine.

Dans un mode de réalisation, l'invention concerne un inhibiteur destiné à être utilisé tel qu'indiqué précédemment, caractérisé en ce qu'il s'agit d'un inhibiteur nucléosidique choisi parmi l'azidothymidine (AZT ou Zidovudine), la 2'-3'-dideoxycytidine (ddC ou Zalcitabine), le [(1R)-4-[2-amino-6-(cyclopropylamino)purin-9-yl]-1-cyclopent-2-enyl]methanol (ABC ou Abacavir), la 2'-3'-didehydro-2'-3'-dideoxythymidine (d4T ou Stavudine), la 2',3'-dideoxy-3'-thiacytidine (3TC ou Lamivudine), la ddI (2'-3'-dideoxyinosine), le 4-amino-5-fluoro-1-[2-(hydroxymethyl)-1,3-oxathiolan-5-yl]- pyrimidin-2-one (FTC), le 2-(6-aminopurin-9-yl)ethoxymethyl-phosphonic acid (bis-POM PMPA), l'Adefovir, la Didanosine, l'Emtricitabine et le Ténofovir.

Dans un mode de réalisation, l'invention concerne un inhibiteur destiné à être utilisé tel qu'indiqué précédemment, caractérisé en ce que ledit inhibiteur est la Stavudine.

La présente description a pour objet un inhibiteur de transcriptase inverse pour son utilisation dans la prévention et/ou le traitement d'une maladie dégénérative.

Conformément à l'invention, la maladie dégénérative est une maladie d'origine génétique et/ou acquise, liée notamment à l'âge (vieillissement) et/ou au stress, notamment au stress oxydatif.

Du fait qu'il réduit la formation de DSB, l'inhibiteur de transcriptase inverse a un effet thérapeutique dans les dégénérescences liées à l'âge et/ou au stress, ou bien encore à des conditions qui augmentent les effets délétères du stress (une mutation, par exemple).

Il est utile, notamment dans la prévention et le traitement des maladies neurodégénératives, en particulier à déclenchement tardif, du vieillissement neuronal et des effets du stress oxydatif dans les cellules nerveuses ou d'autres types de cellules.

Parmi les maladies dégénératives on peut citer de façon non-limitative les maladies neurodégénératives telles que par exemple, la maladie de Parkinson, la maladie d'Alzheimer, la maladie d'Huntington, la sclérose latérale amyotrophique (SLA), les maladies dégénératives affectant la vue ou l'audition, notamment le glaucome.

L'inhibiteur de transcriptase inverse est un inhibiteur spécifique de la transcriptase inverse. L'inhibiteur de transcriptase inverse est nucléosidique ou non-nucléosidique.

Parmi les inhibiteurs utilisables dans le cadre de la présente invention on peut citer notamment ceux actuellement disponibles comme médicament dans le traitement de l'infection par le Virus de l'Immunodéficience Humaine (VIH), à savoir, les inhibiteurs nucléosidiques tels que : azidothymidine (AZT ou Zidovudine), 2'-3'-dideoxycytidine (ddC ou Zalcitabine), [(1R)-4-[2-amino-6-(cyclopropylamino)purin-9-yl]-1-cyclopent-2-enyl]methanol (ABC ou Abacavir), 2'-3'-didehydro-2'-3'-dideoxythymidine (d4T ou Stavudine), 2',3'-dideoxy-3'-thiacytidine (3TC ou Lamivudine), ddl (2'-3'-dideoxyinosine), 4-amino-5-fluoro-1-[2-(hydroxymethyl)-1,3-oxathiolan-5-yl]-pyrimidin-2-one (FTC), 2-(6-aminopurin-9-yl)ethoxymethyl-phosphonic acid (bis-POM PMPA), Adefovir, Didanosine, Emtricitabine et Ténofovir ; les inibiteurs non-nucléosidiques tels que : Efavirenz (EFV), Névirapine (NVP), Delavirdine (DLV), Etravirine et Rilvipirine.

Selon un mode de réalisation avantageux de l'invention, ledit inhibiteur est un inhibiteur nucléosidique, de préférence la Stavudine.

L'inhibiteur est administré par une voie adaptée à la pathologie à traiter (orale, parentérale, locale) et à des doses suffisantes pour obtenir l'effet thérapeutique désiré, qui peuvent être déterminées aisément par l'Homme du métier.

Outre les dispositions qui précèdent, l'invention comprend encore d'autres dispositions qui ressortiront de la description qui va suivre, qui se réfère à des exemples de mise en œuvre de la présente invention.

### Exemple 1 : Le stress oxydatif induit la formation de cassures double-brin (DSB), une déstructuration de l'hétérochromatine et l'expression des gènes d'éléments transposables (LINEs)

### 1. Matériels et méthodes

### Animaux

Les souris ont été traitées conformément aux directives pour le maintien et l'utilisation des animaux de laboratoire du National Institute of Health (États-Unis) et de la Directive Européenne 86/609 du Conseil CEE pour la protection des animaux utilisés à des fins de recherche expérimentale et autres utilisations scientifiques. Les souris Swiss OF1 de souche sauvage (Janvier) ou mutante hétérozygote *En1+*/*-* (Hanks *et al.,* 1995) ont été maintenues dans des animaleries conventionnelles. Les groupes expérimentaux sont constitués de souris âgées de 6 à 9 semaines.

### Traitement 6-OHDA

Pour les injections de 6-OHDA (6-hydroxydopamine), des souris anesthésiées ont été placées sur un dispositif stéréotaxique et un trou de trépan a été percé dans le crâne à 3.3 mm en caudale et 1 mm en latéral du bregma. L'aiguille de la seringue a été alignée sur le trou et descendue à 4 mm de la surface du crâne et l'injection de 6-OHDA (2 µl; 0.8 µg/µl Sigma) ou du contrôle (Nacl 0,9 %) dans la SNpc a été réalisée pendant plus de 4 min. Pour l'analyse immunohistologique, les souris ont été sacrifiées 6h, 24h ou 7 jours après l'injection de 6-OHDA. Pour les analyses qRT-PCR, des tissus de la SNpc 6h après l'injection 6-OHDA ont été obtenus en réalisant des carottes de 1 mm dans des couronnes de tissu congelé de 2 mm d'épaisseur, à l'aide d'un appareil stéréotaxique.

### qRT-PCR

L'ARN total RNA de tissue de la SNpc a été extrait à l'aide du Kit RNeasy^{®} Lipid Tissue kit (Qiagen) suivi d'une digestion à la DNase I (Thermo), puis une transcription inverse a été réalisée sur 200 ng d'ARN en utilisant le kit Sensiscript^{®} ou QuantiTect Reverse Transcription (Qiagen). Des réactions de qRT-PCR on été effectuées avec des paires d'amorces spécifiques des séquences à amplifier, à l'aide du kit SYBR-Green (Invitrogen ou Roche Applied Science) et un thermocycleur LightCycler^{®} 480 et les valeurs ont été normalisées par rapport aux taux d'ARNm de *Gadph* et/ou *Hprt.* Les résultats ont été analysés à l'aide de la méthode ddCt (Livak and Schmittgen, 2001).

### Immunomarquage

Les souris ont été anesthésiées puis une perfusion transcardiaque a été effectuée avec du PBS puis du PBS contenant 4% de paraformaldéhyde. Les cerveaux ont été post-fixés pendant une 1h et cryoprotégés avec une solution de sucrose 20%.

Les tissus ont été inclus dans l'OCT, congelés dans l'isopentane refroidi, puis conservés à -80°C avant d'être coupés. Des coupes de 20 µm d'épaisseur du cerveau au niveau de la SNpc ont été réalisées. Pour l'immunofluorescence, les lames ont été séchées à l'air, perméabilisées pendant 20 min dans du PBS contenant 1% Triton X-100 et incubées à 100°C pendant 20 min dans du tampon citrate (10 mM acide citrique, 0,05% Tween 20, pH 6,0) pour démasquer l'antigène. Après 1h de blocage (10% sérum normal de chèvre , 0,05% Triton X-100 dans PBS), les tissus ont été incubés toute la nuit à 4°C avec des anticorps primaires dilués dans la solution de blocage (anti-γ-H2AX de souris 1:200, Millipore; anti-TH de poulet, 1:500, Abcam; anti-caspase3 activée de lapin, 1:200, Abcam; anti-nucléoline de lapin, 1:200, Sigma; anti-fibrillarine de lapin, 1:200, Gentex; anti-H3k27me3 de lapin, 1:200, Millipore; anti-H3k9me3 de lapin, 1:200, from Edith Heard; anti-Mecp2 de lapin, 1:300, Abcam; anti-PCNA de lapin, 1:200, Cell Signaling; anti-cycline A de lapin, 1:200, Santa Cruz; anti-phospho-H3 de souris, 1:100, Cell Signaling ; anti-Lamin de lapin, Santa Cruz ; anti-LINE ORF1p, 1:500). Les coupes ont été incubées avec les anticorps secondaires appropriés (488 anti-poulet, 647 anti-poulet, 488 anti-souris, 546 anti-souris and 546 anti-lapin Alexa Fluor, Life technologies) pendant 1h à température ambiante. Les coupes de cerveau marquées ont été visualisées à l'aide d'un microscope confocal à fluorescence (SP5, Leica). Pour l'immunohistochimie TH, les lames ont été perméabilisées dans une solution de 1% Triton X-100 et incubées toute la nuit à 4°C avec du PBS, 10% sérum normal de chèvre contenant un anticorps polyclonal de lapin anti-TH (1:1000; Pel-Freez Biologicals). Les coupes ont été traitées avec un anticorps secondaire biotinylé (Vector Lab) puis incubées avec un complexe avidine-peroxydase de raifort (*horseradish peroxidase ou HRP*) biotinylée (ABC system, Vectastain). La peroxydase a été révélée à l'aide du kit de substrat de peroxydase (HRP) basé sur la diaminobenzidine (DAB) (Vector lab) puis visualisée avec un microscope Eclipe i90 (Nikon).

### Quantification des images

Les images de microscopie ont été analysées à l'aide du logiciel ImageJ. Pour l'immunofluorescence, toutes les quantifications ont été effectuées à l'aide d'un grossissement 60X et des plans focaux successifs de 0,7 µm d'épaisseur. Le marquage au DAPI a été utilisé pour localiser les noyaux des cellules immunomarquées avec la TH. 100 à 300 cellules individuelles TH positives ont été quantifiées pour chaque condition. Pour le comptage des foyers γ-H2AX, le nombre de points γ-H2AX dans le noyau de chaque cellule a été déterminé ; du fait d'un marquage endogène, un seuil de 2 foyers par cellules a été établi. Pour l'analyse des motifs H3K27me3 et Nucléoline, un graphique en deux dimensions de l'intensité des pixels le long d'une ligne positionnée au travers du noyau a été créée. Les motifs spécifiques identifiés ont été utilisés pour déterminer la présence de H3K27me3. Pour l'analyse des marquages H3K9me3 et Mecp2, la même procédure a été utilisée avec les chromocentres DAPI-denses. Le rapport des fluorescences H3k27me3 périnucleaire et nucléaire a été déterminé en mesurant la densité de pixel le long de l'enveloppe nucléaire et du nucléoplasme marqué au DAPI.

### 2. Résultats

Un stress oxydatif mimant un vieillissement accéléré a été induit spécifiquement dans les neurones mésencéphaliques dopaminergiques (mDA) de la substance noire pars compacta (SNpc) en injectant localement une drogue oxydante, la 6-OHDA (6-hydroxydopamine), capturée via les transporteurs à la dopamine (DA) exprimés spécifiquement par les neurones mDA de la SNpc.

Des sections de mésencéphale de souris injectés avec la 6-OHDA et de souris contrôle ont été marquées avec des anticorps spécifiques de γ-H2AX, H3K27me3 et Tyrosine hydroxylase (TH ; un marqueur des neurones dopaminergiques) et analysées en microscopie confocale en fluorescence.

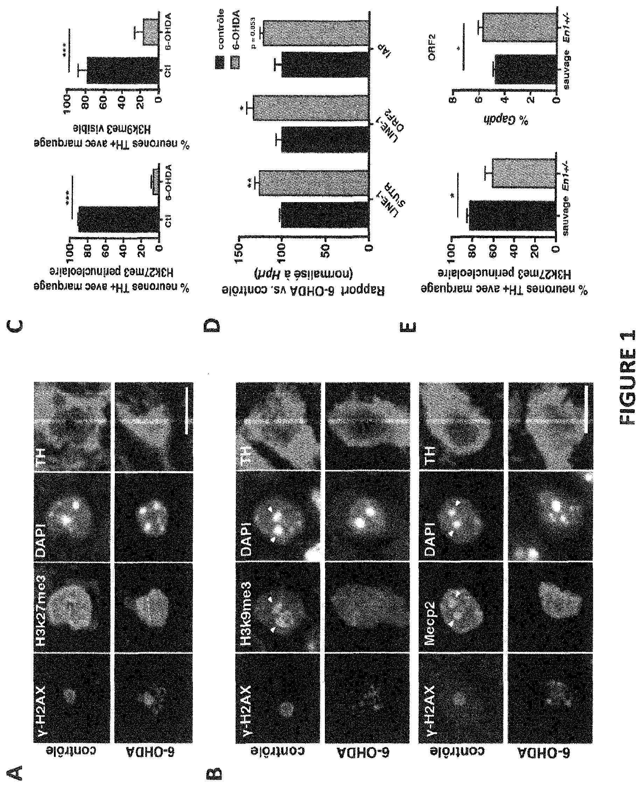

Six heures après l'injection de 6-OHDA dans la substance noire pars compacta (SNpc) des souris, on observe une diminution du marquage H3K27m3 périnucléolaire et périnucléaire dans les neurones TH+ par rapport aux souris contrôles **(****Figure 1A****)**.

Le triple immunomarquage des sections de mésencéphale par γ-H2AX, H3K27me3 et TH montre que la colocalisation de H3K27me3 avec le DAPI observé dans les neurones TH+ des souris contrôle disparaît dans les souris injectées avec la 6-OHDA **(****Figure 1B****).**

De même, la colocalisation de MeCP2 avec le DAPI observé dans les neurones TH+ des souris contrôle disparaît dans les souris injectées avec la 6-OHDA **(****Figure 1B****).**

Le pourcentage de neurones TH+ avec un marquage H3K27me3 périnucléolaire ou H3K9me3 visible est diminué de façon significative dans les souris injectées avec la 6-OHDA (n = 3, *** p<0.001). Le nombre de neurones compté pour le contrôle et 6-OHDA est de respectivement, 148 et 91 pour H3K27me3 et 161 et 97 pour H3K9me3 **(****Figure 1C****)**.

Les taux de transcrits LINE-1 et IAP (pour *Intracisternal A Particle*) dans la SNpc, analysés par qRT-PCR, sont augmentés chez les souris injectées avec la 6-OHDA, par rapport aux animaux contrôles (n=3) **(****Figure 1D****).**

Des sections de mésencéphale de souris *En1* + /- âgées de 9 semaines ont été analysées pour le marquage H3K27. La quantification a montré une diminution du pourcentage de neurones TH+ avec un marquage H3K27 périnucléolaire dense par rapport aux souris sauvages (wt) **(****Figure 1E** ; n = 3, * p<0.05, 125 et 162 neurones comptés chez wt et *En1*+/-, respectivement). Les taux de transcrits LINE-1 ORF2 dans la SNpc de souris *En1*+/- analysés par qRT-PCR augmentent par comparaison aux souris sauvages **(****Figure 1E****;** n = 3-5, * p<0.05 , ** p < 0.01).

La 6-OHDA tue les neurones en 24h via un mécanisme apoptotique (expression de la caspase 3 activée) qui implique la formation de cassures double-brin dans l'ADN (marqueur histone H2AX phosphorylée en S140 (gamma-H2AX)), un stress nucléolaire (dissolution de la nucléoline et de la fibrillarine) et une destructuration de l'hétérochromatine (diffusion de H3K27me3, histone H3 triméthylée sur la lysine 27, un marqueur de l'hétérochromatine), H3K9me3 (histone H3 triméthylée sur la lysine 9), MeCP2 (Methyl-CpG-binding **protein** 2) et Lamine B2, comme illustré aux **Figures 1A, 1B et 1C****.**

Les cassures double-brin de l'ADN et la déstructuration de l'hétérochromatine sont accompagnées de l'expression de gènes normalement réprimés (encodés dans l'hétérochromatine), en particulier des gènes codant des rétrotransposons (dont les Long interspersed Nuclear elements ou LINEs) et des protéines du cycle cellulaire (Cycline A, pH3 et PCNA), normalement silencieux dans ces cellules post-mitotiques, comme illustré à la **Figure 1D****.**

Ces phénotypes sont observables, à un moindre degré chez un mutant Engrailed 1 +/- (*En1*+/-) dont les neurones meurent de façon progressive **(****Figure 1E**).

En conséquence, la **Figure 1** montre que les ROS ou la perte d'un allèle du gène *Engrailed 1* (*En1*) relâche l'hétérochromatine et conduit à l'expression des LINE.

### Exemple 2 : La formation de DSB induite par le stress oxydatif est diminuée en présence d'un inhibiteur de la transcriptase inverse.

### 1. Matériels et Méthodes

### Essais in vitro

### Culture de cellules

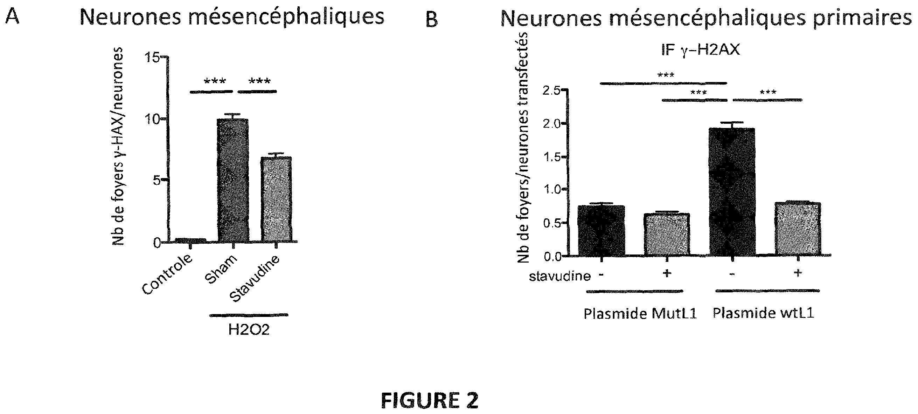

Les neurones embryonnaires de mésencéphale (jour embryonnaire 13,5) sont cultivés dans du milieu NBGK (Neurobasal^{®} (Life Technologies) supplémenté en glutamine (500 µM, SIGMA), acide glutamique (3,3 mg/mL, SIGMA), acide aspartique (3,7 mg/mL, SIGMA), anti-anti et B27^{®} (GIBCO)). Une culture de neurones de mésencéphale a été incubée une nuit en présence de Stavudine (10 µM dans NaCl 0,9 %). Une culture contrôle a été traitée dans les mêmes conditions, avec une solution de NaCl (0,9 %). Le lendemain, le milieu de culture a été remplacé par du milieu Neurobasal^{®} (Life Technologies) non supplémenté en B27^{®} (Life Technologies), contenant 5 µM d'H₂O₂ et 10 µM Stavudine/NaCl, pendant 1h (Figure 2). Alternativement, les cellules ont été traitées avec H₂O₂ (100 µM) en présence de B27 pendant une heure. La Stavudine (10 µM) a été ajoutée deux fois, 24 heures avant et pendant le traitement avec H₂O₂ ou la lipofection (Figure 2). Les neurones ont ensuite été fixés avec du PBS 4% paraformaldéhyde (PFA) pendant 20 min, puis traitées avec de la glycine (100 mM) dans du PBS pendant 10 min. Les cellules ont été bloquées dans du PBS contenant 10 % de sérum de chèvre pendant 1 heure, puis incubées une nuit à température ambiante avec des anticorps primaires anti-histone H2AX phosphorylée en S140 (gamma-H2AX) dans du PBS contenant 3% sérum de chèvre et 0,1 % Triton X-100, et 1 heure à température ambiante avec l'anticorps secondaire couplé à un fluorophore. Le nombre de foyers gamma-H2AX par neurone a été compté sur plusieurs lamelles puis la moyenne a été effectuée sur plus de 30 neurones. Les résultats sont représentatifs de 4 expériences indépendantes.

Le protocole de transfection a été adapté de Dalby et al. (2004). Les plasmides (0,75 µg par transfection) ont été pré-incubés avec 8µL de Lipofectamine^{®} 2000 (Life Technologies) pendant 20 min à température ambiante dans du milieu Opti-MEM^{®} (Life Technologies). Le milieu a été ajouté et le mélange a été ajouté aux cellules pendant 48 h à 37°C, puis l'immunofluorescence a été effectuée.

### Essai de transcription inverse

Des cellules HEK normales ou inductibles par la protéine Engrailed (En) ont été traitées avec de la doxycycline pendant une journée pour induire l'expression d'Engrailed. Ensuite, les cellules ont été transfectées avec un plasmide de rétrotransposition semblable à celui décrit dans Xie et al., 2011, avec seulement un gène LINE-1 de souris et une cassette GFP. Les cellules ont été divisées un jour après la transfection, puis traitées avec de la puromycine (0,7 µg/µl Sigma), 3 jours plus tard, pour éliminer les cellules non-transfectées. Après une semaine, le pourventage de cellules GFP positives a été mesuré par cytométrie de flux.

### Essais in vivo

Des souris ont été traitées à la Stavudine (10 µM ; SIGMA) suivi 30 min plus tard de l'injection d'un mélange contenant la 6-OHDA (2 µl; 0.5 µg/µl ; Sigma) et la Stavudine (10 µM ; SIGMA) comme indiqué à l'exemple 1. L'immunomarquage a été réalisé comme décrit à l'exemple 1 puis visualisé avec un microscope Optiphot 2 (Nikon). Les images de microscopie ont été analysées à l'aide du logiciel VisioScan T4.18 (ExploraNova, La Rochelle, France) comme décrit précédemment (Höglinger et al. 2003). Les nombres de cellules ont été quantifiées de façon stéréologique sur des sections espacées de façon régulière couvrant la totalité de l'étendue rostro-caudale de la substance noire avec l'outil de stéréologie VisioScan. La substance noire pars compacta (SNpc) a été identifiée selon des repères anatomiques établis (Atlas du cerveau de souris de Paxinos). L'analyse qRT-PCR a été réalisée comme décrit à l'exemple 1, en utilisant les paires d'amorces suivantes: la paire SEQ ID NO : 1 et 2 (LINE-1 Tf/Gf) et la paire SEQ ID NO : 3 et 4 (LINE-1A), spécifiques du gène LINE-1 ; la paire SEQ ID NO : 5 et 6, spécifique du gène *Hprt ;* la paire SEQ ID NO : 7 et 8, spécifique du gène *Gapdh.*

### 2. Résultats

Des neurones de mésencéphale d'embryon de souris (14 jours de gestation), traités ou non avec un inhibiteur de RT (Stavudine), ont été soumis à un stress oxydatif par addition de H₂O₂. Ce superoxide, contrairement à la 6-OHDA n'est pas spécifique des seuls neurones mDA (qui ne comptent que pour 1,5 % dans la culture) mais affecte toutes les cellules. Le nombre de DSB par neurone a été évalué après marquage γ-H2AX.

Les résultats présentés dans la **Figure 2A** montrent que H₂O₂ augmente le nombre de DSB *in vitro.* Toutefois, la formation de DSB est diminuée de façon significative (réduction de 40 %) par la Stavudine, un inhibiteur de transcriptase inverse qui diminue la formation des cassures de l'ADN à partir des transcrits LINE (***p<0,001; n=6, Test ANOVA à un facteur, Test de comparaison multiple de Bonferroni). La **Figure 2A** montre donc qu'un inhibiteur de RT diminue la formation de DSB induits par un stress oxydatif *in vitro.*

La transfection d'un plasmide (wt L1) surexprimant le gène LINE-1 dans des neurones primaires du mésencéphale induit la formation de cassures de l'ADN (Figure 2B). Cet effet est réprimé par la Stavudine **(****Figure 2B****)** ou un plasmide (Mut L1) dans lequel l'ORF2 du gène LINE-1 est mutée (***p<0,001; n=6, Test ANOVA à un facteur, Test de comparaison multiple de Bonferroni).

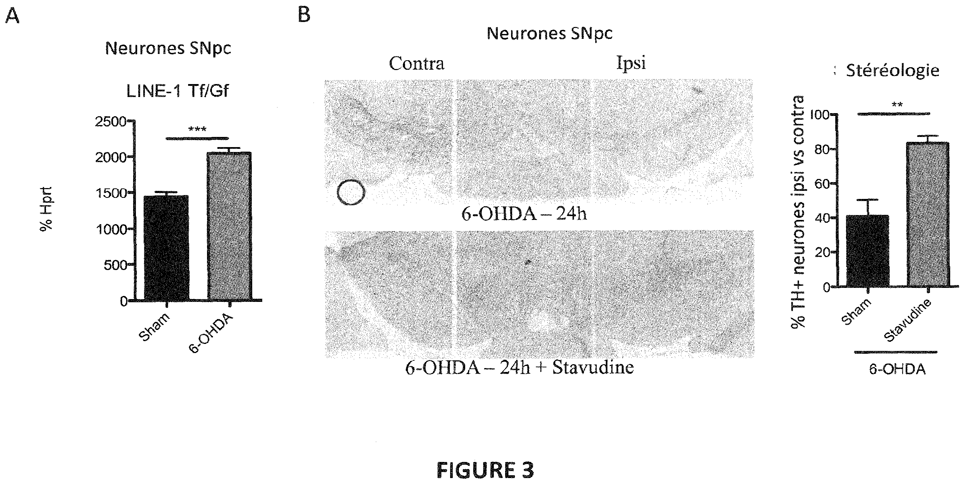

L'injection de 6-OHDA dans la substance noire qui mime un stress oxydatif *in vivo* montre une augmentation de la transcription des LINE (LINE-1 Tf) dans les neurones de la substance noire pars compacta (SNpc ; **Figure 3A****).**

Le nombre de neurones TH+ de la substance noire pars compacta, 24 h après l'injection de 6-OHDA, est plus élevé chez les souris traités avec la Stavudine **(****Figure 3B** ; **p<0,01, n=5, test de Student). Ces résultats démontrent que l'injection de Stavudine diminue la mort cellulaire après un stress oxydatif *in vivo.*

### LISTE DES REFERENCES

1. Hanks, M., Wurst, W., Anson-Cartwright, L., Auerbach, A.B., and Joyner, A.L. (1995). Rescue of the En-1 mutant phenotype by replacement of En-1 with En-2. Science 269, 679-682.
2. Livak, K.J. and Schmittgen, T.D. (2001). Analysis of relative gene expression using real-time quantitative PCR and the 2(-Delta Delta C(T)) Method 4, 402-408.
3. Marteijn, J.A., Lans, H., Vermeulen, W., and Hoeijmakers, J.H. (2014). Understanding nucleotide excision repair and its roles in cancer and ageing. Nat Rev Mol Cell Biol 15, 465-481.
4. O'Sullivan, R.J., and Karlseder, J. (2012). The great unravelling: chromatin as a modulator of the aging process. Trends Biochem Sci 37, 466-476.
5. Suberbielle, E., Sanchez, P.E., Kravitz, A.V., Wang, X., Ho, K., Eilertson, K., Devidze, N., Kreitzer, A.C., and Mucke, L. (2013). Physiologic brain activity causes DNA double-strand breaks in neurons, with exacerbation by amyloid-beta. Nat Neurosci 16, 613-621.
6. Vijg, J., and Suh, Y. (2013). Genome instability and aging. Annu Rev Physiol 75, 645-668.
7. Höglinger, G.U. et al., 2003. Chronic systemic complex I inhibition induces a hypokinetic multisystem degeneration in rats. Journal of neurochemistry, 84(3), pp.491-502.
8. Xie, Y. et al., 2011. Characterization of L1 retrotransposition with high-throughput dual-luciferase assays. Nucleic Acids Research, 39(3), pp.e16-e16.
9. Dalby, B. et al., 2004. Advanced transfection with Lipofectamine 2000 reagent: primary neurons, siRNA, and high-throughput applications. Methods (San Diego, Calif.), 33(2), pp.95-103.

## Revendications

1. Inhibiteur de transcriptase inverse destiné à être utilisé dans la prévention ou le traitement d'une maladie dégénérative choisie parmi la maladie de Parkinson et la maladie d'Alzheimer, ledit inhibiteur de transcriptase inverse étant choisi parmi :
- les inhibiteurs nucléosidiques,
- l'Efavirenz (EFV),
- la Névirapine (NVP),
- la Delavirdine (DLV),
- l'Etravirine, et
- la Rilvipirine.

2. Inhibiteur destiné à être utilisé selon la revendication 1, **caractérisé en ce qu'**il s'agit d'un inhibiteur nucléosidique choisi parmi l'azidothymidine (AZT ou Zidovudine), la 2'-3'-dideoxycytidine (ddC ou Zalcitabine), le [(1R)-4-[2-amino-6-(cyclopropylamino)purin-9-yl]-1-cyclopent-2-enyl]methanol (ABC ou Abacavir), la 2'-3'-didehydro-2'-3'-dideoxythymidine (d4T ou Stavudine), la 2',3'-dideoxy-3'-thiacytidine (3TC ou Lamivudine), la ddl (2'-3'-dideoxyinosine), le 4-amino-5-fluoro-1-[2-(hydroxymethyl)-1,3-oxathiolan-5-yl]-pyrimidin-2-one (FTC), le 2-(6-aminopurin-9-yl)ethoxymethyl-phosphonic acid (bis-POM PMPA), l'Adefovir, la Didanosine, l'Emtricitabine et le Ténofovir.

3. Inhibiteur destiné à être utilisé selon l'une quelconque des revendications 1 à 2, **caractérisé en ce que** ledit inhibiteur est la Stavudine.

## Patentansprüche

1. Reverse-Transkriptase-Inhibitor, zur Verwendung bei der Vorbeugung oder der Behandlung einer degenerativen Krankheit, ausgewählt aus der Parkinson-Krankheit und der Alzheimerkrankheit, wobei der Reverse-Transkriptase-Inhibitor ausgewählt ist aus:
- nukleosidischen Inhibitoren,
- Efavirenz (EFV),
- Nevirapin (NVP),
- Delavirdin (DLV),
- Etravirin und
- Rilvipirin.

2. Inhibitor, zur Verwendung nach Anspruch 1, **dadurch gekennzeichnet, dass** es sich um einen nukleosidischen Inhibitor handelt, ausgewählt aus Azidothymidin (AZT oder Zidovudin), 2'-3'-Dideoxycytidin (ddC oder Zalcitabin), [(1 R)-4-[2-Amino-6-(cyclopropylamino)purin-9-yl]-1-cyclopent-2-enyl]methanol (ABC oder Abacavir), 2'-3'-Didehydro-2'-3'-dideoxythymidin (d4T oder Stavudin), 2',3'-Dideoxy-3'-thiacytidin (3TC oder Lamivudin), ddl (2'-3'-Dideoxyinosin), 4-Amino-5-fluor-1-[2-(hydroxymethyl)-1,3-oxathiolan-5-yl]-pyrimidin-2-on (FTC), 2-(6-Aminopurin-9-yl)ethoxymethyl-phosphonsäure (Bis-POM PMPA), Adefovir, Didanosin, Emtricitabin und Tenofovir.

3. Inhibitor, zur Verwendung nach einem der Ansprüche 1 bis 2, **dadurch gekennzeichnet, dass** der Inhibitor Stavudin ist.

## Claims

1. Reverse-transcriptase inhibitor for use in the prevention or the treatment of a degenerative disease chosen from Parkinson's Disease and Alzheimer's Disease, said reverse-transcriptase inhibitor being chosen from:
- nucleoside inhibitors,
- Efavirenz (EFV),
- Nevirapine (NVP),
- Delavirdine (DLV),
- Etravirine, and
- Rilviprine.

2. Inhibitor for use according to claim 1, **characterised in that** it is a nucleoside inhibitor chosen from azidothymidine (AZT or Zidovudine), 2'-3'-dideoxycytidine (ddC or Zalcitabine), [(1R)-4-[2-amino-6-(cyclopropylamino)purin-9-yl]-1-cyclopent-2-enyl]methanol (ABC or Abacavir), 2'-3'-didehydro-2'-3'-dideoxythymidine (d4T or Stavudine), 2'-3'-dideoxy-3'-thiacytidine (3TC or Lamivudine), ddl (2'-3'-dideoxyinosine), 4-amino-5-fluoro-1-[2-(hydroxymethyl)-1,3-oxathiolan-5-yl]-pyrimidin-2-one (FTC), 2-(6-aminopurin-9-yl)ethoxymethyl-phosphonic acid (bis-POM PMPA), Adefovir, Didanosine, Emtricitabine and Tenofovir.

3. Inhibitor for use according to any one of claims 1 to 2, **characterised in that** said inhibitor is Stavudine.


### SEQUENCE LISTING

<110> CENTRE NATIONAL DE LA RECHERCHE SCIENTIFIQUE INSTITUT NATIONAL DE LA SANTE ET DE LA RECHERCHE MEDICALE COLLEGE DE FRANCE
<120> UTILISATION D'UN INHIBITEUR DE TRANSCRIPTASE INVERSE DANS LA PREVENTION ET LE TRAITEMENT DES MALADIES DEGENERATIVES
<130> F644PCT375
<150> FR1460535
   <151> 2014-10-31
<160> 8
<170> PatentIn version 3.5
<210> 1
   <211> 20
   <212> DNA
   <213> artificial sequence
<220>
   <223> synthetic primer
<400> 1
   ctgggaactg ccaaagcaac 20
<210> 2
   <211> 20
   <212> DNA
   <213> artificial sequence
<220>
   <223> synthetic primer
<400> 2
   cctccgttta cctttcgcca 20
<210> 3
   <211> 20
   <212> DNA
   <213> artificial sequence
<220>
   <223> synthetic primer
<400> 3
   ttctgccagg agtctggttc 20
<210> 4
   <211> 20
   <212> DNA
   <213> artificial sequence
<220>
   <223> synthetic primer
<400> 4
   tgagcagacc tggagggtag 20
<210> 5
   <211> 22
   <212> DNA
   <213> artificial sequence
<220>
   <223> synthetic primer
<400> 5
   agcaggtgtt ctagtcctgt gg 22
<210> 6
   <211> 22
   <212> DNA
   <213> artificial seqeunce
<220>
   <223> synthetic primer
<400> 6
   acgcagcaac tgacatttct aa 22
<210> 7
   <211> 21
   <212> DNA
   <213> artificial sequence
<220>
   <223> synthetic primer
<400> 7
   tgacgtgccg cctggagaaa c 21
<210> 8
   <211> 21
   <212> DNA
   <213> artificial sequence
<220>
   <223> synthetic primer
<400> 8
   ccggcatcga aggtggaaga g 21